Europäisches Patentamt

European Patent Office

Office européen des brevets

Publication number: **0 283 613**

**A2**

# EUROPEAN PATENT APPLICATION

Application number: **87303878.0**

Date of filing: **30.04.87**

Int. Cl.⁴ **G01N 33/52** , **C12Q 1/00** , **C12Q1/54,G01N33/545**

The title of the invention has been amended (Guidelines for Examination in the EPO, A-III. 7.3).

Priority: **25.02.87 US 18453**

Date of publication of application: **28.09.88 Bulletin 88/39**

Designated Contracting States: **BE DE ES FR GB IT LU NL SE**

Applicant: **GENESIS LABS, INC.**
**5182 W. 76th St.**
**Edina Minnesota 55435(US)**

Inventor: **Smith, Kelly D.**
**2733 S. Gerard B2**
**Minneapolis Minnesota 55408(US)**
Inventor: **Maddox, Catherine B.**
**1480 E. Brooks Ave.**
**St.Paul Minnesota 55109(US)**

Representative: **Bond, Bentley George et al**
**HASELTINE LAKE & CO. Hazlitt House 28**
**Southampton Buildings Chancery Lane**
**London, WC2A 1AT(GB)**

**Dry test strip suitable for oxygen demanding detection system.**

Immunoassay and chemical assay dry test strips for the detection of a target analyte in a test fluid using a dry chemistry detection means requiring atmospheric oxygen for appropriate analyte detection comprises a detection zone supported on a carrier strip and optionally protected by a protective film wherein the film and the carrier strip can have an aperture means for providing to the chemistry a sufficient supply of atmospheric oxygen for appropriate analyte detection.

FIG. IA  FIG. IB  FIG. IC

## DRY TEST STRIP FOR DEVICES USING OXYGEN DEMANDING DETECTION SYSTEM

### Field of the Invention

The invention relates to a dry test strip for an immunoassay or chemical assay, useful in the detection of clinically significant analytes in biological or other test fluids. Particularly the invention relates to a dry test strip having an immunological or chemical detection system requiring oxygen for analyte detection, having a particular test strip format that insures sensitivity, precision and accuracy through the availability and rapid delivery of oxygen to the detection system.

### Background of the Invention

In recent years, dry test strips have been used to a considerable extent in the analysis, especially in clinical chemistry and in at-home monitoring of blood levels of a variety of analytes including glucose. Dry test strips are typically in the form of a carrier strip having an absorbent layer impregnated with reagents that can detect and identify the presence of a target analyte in a test fluid containing the analyte. The presence of target analyte is typically signaled by the development of a color, the production of a color change, or the production of a fluorescent or other electromagnetic irradiation associated signal from a surface of the dry test strip.

A common type of signal generating system used in both chemical analysis and immunoassay analysis involves signal detection systems requiring atmospheric oxygen for proper operation. Representative examples of dry test strips that can be used in detection of analytes with reagent systems requiring atmospheric oxygen are found in Lang et al, U.S. Pat. No. 4,061,468, Fenocketti et al, U.S. Pat. No. 4,160,008, and Cowsar et al, U.S. Pat. No. 4,181,500 and in commercially available dry test strips marketed under the name CLINISTIX®. Such dry test strips are characterized by a plastic film holding strip which acts as a base layer supporting the detection system in combination with absorbent materials containing the reagent system in many formats. The reagent system can be contained in multiple zones within an absorbent matrix or can be held within a single zone. The entire reagent containing absorbent zone can be covered by other protective layers. In this test strip format, the availability of oxygen in the detection zone can be limited by exclusion of atmospheric oxygen by the plastic film holding strip. Plastic strips typically used in the preparation of the dry test strip devices of the invention are typically not significantly permeable to the passage of atmospheric oxygen. Further, other protective or covering layers applied to the surface of the detection zone can also significantly reduce the amount of atmospheric oxygen available for the detection reaction.

In a preferred embodiment of using the dry test strips of this invention, whole blood is often applied to the dry test strip and the signal produced by the analyte in the whole blood is detected on the side of the test strip opposite to that of the blood application. Accordingly, the color change or other signal is observed through the plastic film holding strip. In the instance that the dry test strip is contacted with whole blood, the lack of atmospheric oxygen at the plastic film holding strip detection zone interface can interfere in the sensitivity, precision and accuracy of the assay.

Accordingly, a substantial need exists for a dry test strip format that insures the ready availability of atmospheric oxygen to the detection zone of the dry test strip.

### Brief Description of the Invention

The dry test strips of the invention are characterized by a flexible carrier strip and a chemical or immunoassay detection zone, adhered thereto, using a reagent system requiring the presence of atmospheric oxygen. The flexible carrier strip has an oxygen aperture for insuring the availability and delivery of an effective color generating concentration of atmospheric oxygen in the reagent zone. The flexible carrier strip can have a single aperture or a plurality of smaller apertures. Further, any protection layer adhered to the detection zone opposite the dry carrier strip can also have an aperture or a plurality of apertures to insure the availability of atmospheric oxygen to the dry test strip.

### Brief Description of Drawings

FIGURE 1 provides views of a dry test strip of the invention showing an oxygen providing aperture in the flexible carrier strip.

FIGURE 2 is an isometric drawing of a dry test strip of the invention showing a plurality of apertures in the flexible carrier strip.

FIGURE 3 is an isometric drawing of the dry test strip of the invention showing an oxygen providing aperture through the protective layer covering the reagent zone which is in turn adhered to the flexible carrier strip which has a separate oxygen providing aperture.

FIGURE 4 is a side cross-sectional view of a dry test strip of the invention showing an oxygen providing aperture in a flexible carrier strip.

FIGURE 5 is a side cross-sectional view of a dry test strip of the invention showing an aperture between the flexible carrier strip and the detection zone of the invention permitting the free flow of atmospheric oxygen to the carrier strip side of the detection zone.

FIGURES 6 and 7 are isometric drawings of a machine readable embodiment of the dry test strip of the invention. The embodiments contain means to produce a blood sample which can be transferred by the devices to the dry test strip for signal generation purposes.

Detailed Discussion of the Invention

The dry test strips of the invention comprise a detection zone, containing a reagent system requiring oxygen to detect the presence of an analyte, adhered to the surface of a flexible carrier strip. The detection zone can further be covered with a flexible film which can adhere to the flexible strip to substantially enclose the detection zone. Both the carrier strip and the protective film can contain one or more oxygen providing apertures to insure that the reagent system providing a detection signal for the presence of the analyte has sufficient oxygen to obtain a sensitive, precise and accurate reading.

In one embodiment the oxygen providing aperture of the dry test strip of the invention can comprise a space formed between the detection zone and the flexible carrier strip providing access to the carrier strip side of the detection zone by atmospheric oxygen. The strip is formed by adhering the detection zone to the carrier strip through small adhesive means that act as both attachment points and as spacers. As a spacer, the adhesive means separates the detection zone from the flexible carrier strip by sufficient distance to permit the easy access to the carrier strip side of the detection zone by atmospheric oxygen. The adhesive means can take the form of thickened dots of adhesive, thickened lines of adhesive, solvent cast adhesive applications, or randomly applied adhesive areas. In order to provide atmospheric access to the carry strip side of the detection zone, a separation of at least 1 mil (1 mil equals 0.001 inches) is required. Preferably for ease of manufacture and use, the separation distance is about 2-10 mils. The adhesive reagents forming the attachment points and spacer functions can be applied as preformed adhesive portions, can be applied as hot melt compositions or can be cast from liquid solution. Typical well known pressure sensitive adhesives can be used.

The flexible carrier strip satisfies a number of uses in the invention. The carrier strip is both a base substrate to which the detection zone is adhered, is typically a holder or handle to provide ease of handling and use, can contain a label or labels for the purpose of identifying the analyte or providing directions; and can provide an oxygen providing aperture. Any flexible material to which the reagent zone can be adhered can be useful as a carrier strip. The carrier strip can be either transparent, translucent, or substantially opaque. The carrier strip can be transparent to enhance the ability of the user to detect any change in the reagent zone indicating the presence of the analyte. The carrier strip must lend itself to machining to obtain the aperture or apertures required for efficient oxygen transfer into the detection zone for the proper detection operation. Such holes can be formed into zones by punching, drilling, or by the initial casting or forming of the carrier strip.

Suitable materials for the preparation of the carrier strip include metal foil, cellulosic materials including paper, insolubilized starch materials, thermoplastic materials, fiberglass, nonwoven fabrics made from both thermoplastic and nonthermoplastic materials, and others. Preferably the carrier strips are made from sheets of thermoplastic materials. Preferred thermoplastic materials include polyvinyl chloride, polyethylene terephthalate, polycarbonate, polyester, nylon, polyethylene, polypropylene, poly-4-methyl-1-butene, polystyrene, high impact polystyrene, acrylonitrile-butadiene-styrene copolymers, silicone rubbers, glass laminates, and others. The thermoplastic films can be colored, can be filled with organic or inorganic components, and can have laminated therein labels or other materials.

The carrier strip can take virtually any outline or shape. The strip can be circular, oval, triangular, rectangular, or any other polygon. Preferably the carrier strip is a rectangle that is approximately 1 to 10 millimeters in width and 25 to 300 millimeters in length. The thickness of the carrier strip is typically kept within 0.15 to 0.5 millimeters (7 to 15 mils (1 mil = 0.001 inch)).

The detection zone is typically adhered to the dry test strip and has an area of adhesion that is about 1 to 20 square millimeters. In order to insure that the detection zone has enough oxygen to insure a rapid, sensitive, precise and accurate detection of the analyte, as much of the detection zone is exposed to atmospheric oxygen as is practically possible. Such exposure is obtained by adhering the detection zone to the carrier strip utilizing as little of the detection zone surface as possible. The detection zone can be adhered to the carrier strip at the extreme periphery of the detection zone or at selected points on the interior surface of the detection zone. Alternatively, if a plurality of apertures is used, the detection zone is adhered to the carrier strip on the periphery of each of the plurality of apertures. The detection zone is adhered to the carrier strip in a manner such that greater than 50% of the area of the detection zone is exposed to atmospheric oxygen, preferably greater than 75% of the area, and most preferably greater than 90%.

The shape of the aperture or each aperture can be independently circular, oval, eliptical, or any polygonal shape. Preferably, for ease of manufacture, the apertures are circular and can have a radius of about 3 to 10 millimeters (3/32″ to 5/32″). The maximum dimension of the radius of each aperture is just less than 1/2 the major dimension of the detection zone. Such a radius will permit the detection zone to adhere to at least two locations on the carrier strip. If the detection zone takes roughly a square or rectangular shape, the detection zone will adhere to the carrier strip at the edge of the aperture. The detection zone of the dry test strip will be roughly rectangular, having dimensions of 1 to 15 millimeters by 1 to 15 millimeters, having a surface area of about 10 to 200 square millimeters. Covering the detection zone is a second film sealing the detection zone. The sealing film can also contain oxygen providing aperture or apertures similar to that in the carrier strip.

The dry test strip device of this invention can be used in a chemical assay or an immunoassay system for the detection of the presence of virtually any analyte of interest. Typical analytes detected by the devices of this invention can be characterized as small molecules, large or macromolecules, cellular components, cell organelles, or viruses or cells.

Typical large analytes detected by the device of this invention are characterized as typically large molecule polypeptides, polysaccharides, polynucleic acids, and combinations thereof. Such large analytes can also include somatic cells, germ cells, bacteria, viruses and other cellular units. Subcellular units which can be detected include viral protein, cell wall polysaccharide, DNA, DNA segments, RNA, transfer RNA, messenger RNA, mitochondrial DNA, mitochondrial cell nuclei, cell membrane, rhibozomes, and other varied cell organelles, subunits and constituent parts. Such large analytes are typically detected using immunological dry test strips of the invention and can have a molecular weight in excess of about 50,000. Many such analytes can have a molecular weight ranging from 50,000 to 5,000,000 and more. The analytical device of the invention can also be used to detect and quantitate the presence of analytes having modest molecular weights, i.e. molecules with a molecular weight less than about 15,000, typically between 5,000 and 50,000. A wide variety of such analytes that comprise natural proteins and protein subunits can be detected using the device of the invention. Such proteins include histones, globulins, nuclear proteins, lipoproteins, glycoproteins, somatotropin, prolactin, insulin, pepsin, human plasma protein constituents including human albumin, thyroxin binding globulin, haptoglobin, ceruloplasmin, cholinesterase, myoglobin, fibrinogen, plasminogen, poly and monoclonal immunoglobulins of the A, D, E, G, or M classes, free, light or heavy chains of immunoglobulins, Fab fragment or $F(ab')_2$ fragment, immunoglobulin regions, compliment, blood clotting factors, peptide and protein hormones such as LH, HCG, vasopresin, and others. Antigenic polysaccharides derived from pathogen cell walls also act as immunological antigen.

Further, small molecules of natural and synthetic origin can also be detected using the dry test strip of the invention. Such molecules can be detected using both chemical and immunological detection schemes. Such small molecules typically have a molecular weight of about 100 to 5,000, typically about 100 to 2,000. Such analytes include small molecule natural biochemicals, ethical and over the counter and illicit drugs, hormones, peptides, monodisaccharides, metabolites, pesticides, pollutants and other organic synthetic chemicals. Drugs of interest include ethanol or alkaloids such as morphine, codeine, heroin, dextramethorphan, derivatives and metabolites. Also included are ergot alkaloids such as LSD, steroid alkaloids, quinoline alkaloids and others. Ethical drugs of interest include steroids, bile acids, digoxin, diethylstylbisterol, ethinylesterdiol, and others. Other drugs include barbiturates such as phenobarbitol, secobarbitol, and others. Additionally drugs such as amphetamines, catecholamines, L-dopa, epineffrin, chlorpromazine, benzodiazapine, phenothiazine, theophylline, caffeine, canabis drugs such as cannibinol, tetrahydrocannibinol, vitamins, prostaglandins, antibiotics such as penicillin and the penicillin variants, cephalosporin, and the cephalosporin variants, chloromycetin, actinomycetin, tetracycline, nucleosides and nucleotides, fragments and derivatives thereof including ATP, NAD, FMN, AZTP, and others. Additionally, drugs including methadone, meprobamate, ceratonin, lidocain, propanolol, antihistamines, anticolinergic drugs, and others can be detected. Further, analytes typically detected in clinical chemistry analysis including glucose, cholesterol, triglycerides, uric acid, urea, and other typical small molecule chemical analytes can also be determined.

The primary function of the materials making up the detection zone is to act as a site or locus for detection of the analyte using an effective concentration of the detection zone reagents and to provide for an effective flow of the test fluid through the zone to permit reaction between the analyte and the reagents. The detection zone format can be selected from a variety of shapes or forms having varied dimensions depending on the immunoassay or chemical assay and the materials used. The typical zone material will have a thickness of at least 1 mil, typically greater than 1 mil, generally in the range of 2 to 30 mils. Such substrate materials can be opaque, semi-opaque, translucent or transparent. However, the signal generated by the reagent in the immuno or chemical assay should not be masked by the nature of the support. Both organic and inorganic materials including natural and synthetic materials can be used in the formation of the detection zone. Examples or organic polymeric materials preferred for the formation of the detection zone include polyethylene, polyvinyl chloride, polypropylene, poly-4-methyl-1-butene, polystyrene, polymethacrylate, polyethylene terephthalate, polyester, rayon, nylon, polyvinyl butyrate, silicone films, cellulose, cellulose acetate, nitrocellulose, composite films, and others. Other materials which can be considered for the detection zone include paper, other cellulosics, glass, fiberglass, ceramics, metals, metal foils, metalloids, semiconductive materials, and others. Additionally natural substances that can form gels or films including proteins, protein derivatives, cellulosics, drying oils, and others can be implemented.

A preferred substrate for forming the reagent containing detection zones of the invention comprises a porous nylon substrate material formed by casting a nylon sheet on a nonwoven synthetic organic substrate layer. Such a layer provides uniform pore size at least 0.02 microns preferably 0.04 to 1 micron, chemical inertness to typical solvents and production chemicals, and reagents used in forming the dry test strips, and further provides significant mechanical strength and integrity that promotes rapid and accurate production and use.

The detection zone can contain a reagent system that can generate a unique signal in the presence of the target analyte of the dry test strip. In the analysis of test fluids containing target analytes such as glucose, alcohol, hemoglobin, cholesterol and others, chemical-enzymatic methods can be used in which the enzymatic reaction utilizes an oxidase coupled with a peroxidase requiring the supply of atmospheric oxygen to generate peroxide which is detected using peroxidaze and a chromophore chemical methods. Enzymes useful in such reactions include lactoperoxidase, glucose oxidase, cholesterol oxidase, alcohol oxidase and others. Such oxygen requiring chemical systems are well known in the art.

Immunoassay reagent systems can also be used in the detection zone of the invention. In such assays, reagents requiring the presence of atmospheric oxygen are coupled to antigen or antibody molecules which using the particular specific immunological binding affinity to specific molecules can be used to generate signals unique for the target analyte. Many immunoassays are known in the art. A particularly useful immunoassay is disclosed in Liotta, U.S. Pat. No. 4,446,232. A test strip utilizing the Liotta type technology of the invention has a detection zone comprising a matrix of three zones, a first labeled reagent zone, a second trapping zone, and a third detection zone for label detection. The first label reagent zone contains labeled antibody or a fragment thereof capable of binding a target analyte. The second trapping zone contains typically bound antigen and the third detection zone contains means for detecting the presence of the label on the antibody or fragment thereof. Such label is part of a detection - scheme requiring the presence of atmospheric oxygen. In the operation of the Liotta type device, a test fluid containing the target analyte is applied to the matrix. The analyte in the fluid binds the labeled antibody. The presence of the analyte on the binding sites of the antibody causes the analyte antibody labeled complex to penetrate the matrix and pass through the trapping zone wherein the presence of the analyte prevents the antibody and its label from becoming trapped in the trapping zone. The protected antibody and label penetrate the third zone wherein the presence of the label is detected. In the absence of the analyte in the test fluid, no antigen can bind to the labeled antibody. As the test fluid causes the unbound labeled antibody to penetrate the second layer, bound antigen reacts with and traps the label antibody in its second layer, preventing any of the label from penetrating and causing a detection signal in the third layer. In this way the presence of the analyte in the test fluid can produce a qualitative or quantitative signal in the detection layer.

The dry test strips of the invention can be used for the detection of analytes in a variety of test fluids containing a variety of analytes. Typical test fluids comprise natural or biological fluids that can contain red blood cells including whole blood, whole blood derivatives, red blood cell suspensions, red blood cell preparations, urine, cerebral spinal fluid, acides, saliva, any other clinically important fluid. In use, a controlled volume of the test fluid is applied to the test strip of the invention. Typically useful volumes range from about 5-500 microliters, preferably 5-100 microliters most preferably about 10-50 microliters.

The dry test strip of the invention can be used both as a qualitative, semi-quantitative, or quantitative analytical tool. As qualitative or semi-quantitative, the dry test strip of the invention can be read by an individual seeking to monitor the concentration of an analyte in his blood serum at home such as glucose. Such monitoring can be read by estimating the concentration of the analyte, by a reading of the depth of the color in the detection zone. Such reading can be done manually by eye, can be done by comparing the depth of the color to a comparison standard chart or the qualitative or semi-quantitative estimation schemes. The depth of color on the detection zone can also be read using an instrumental method obtaining a semi-quantitative or fully quantitative measurement of concentration. Particularly effective methods of monitoring a target analyte such as glucose is disclosed in the Garcia et al United States Patent Nos. 4,627,455 and 4,637,403. The monitoring systems disclosed comprise a package containing a chemical reagent strip, a disposable needle or lance probe to obtain a blood sample, a means to insure the flow of blood to the detection zone held within the holder and means to read the color change of the detection zone with a reflectometer apparatus. In the preferred mode of operating such a monitoring system is used containing a detection system of the invention wherein a lance creates a blood sample from typically a finger. The blood sample contacts a wicking fabric which carries the blood sample to the detection zone and can simultaneously carry atmospheric oxygen. The holder containing the detection zones now contacted by the blood sample is inserted into a machine reader which compares a standard detection zone free of reagent system to the color developed in the detection zone containing the appropriate chemical needs. Such a monitoring system can be used to rapidly and accurately screen for the target analyte in a large patient population. Figures 19 and 20 of Garcia et al, U.S. Patent No. 4,637,403 provide a particularly useful blood analyte monitoring system.

Detailed Discussion of Drawings

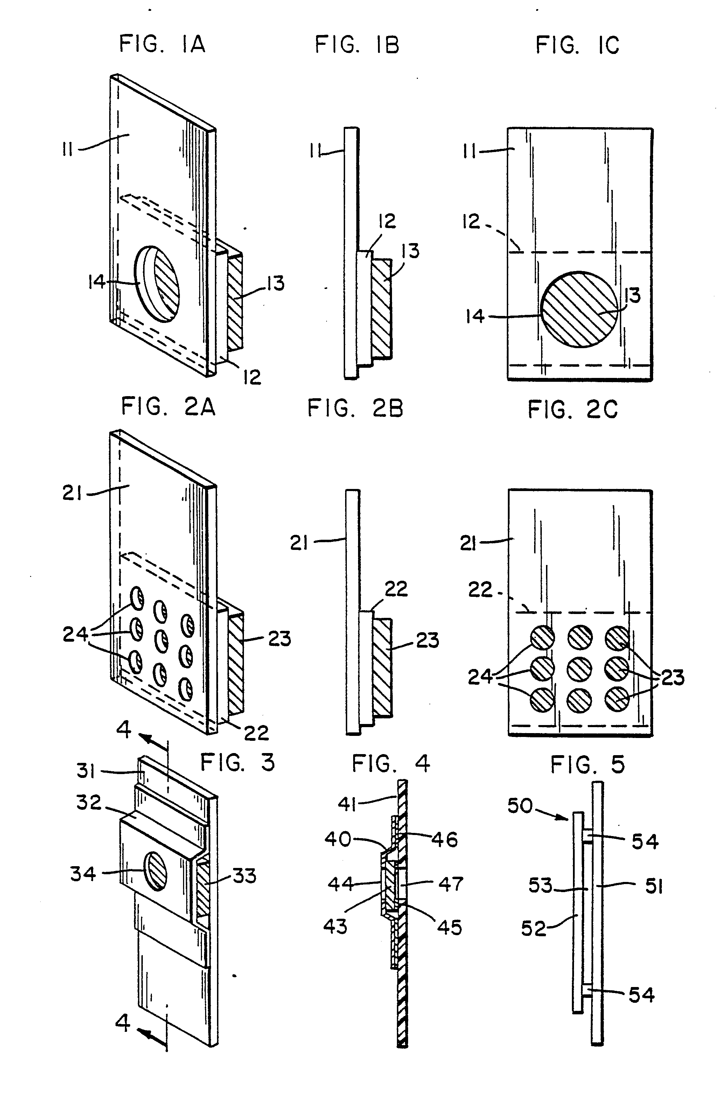

Fig. 1 shows an isometric view of the test strip of the invention. The test device of the invention comprises a thermoplastic flexible carrier strip 11 having a layer of pressure sensitive adhesive 12. The pressure sensitive adhesive bonds the detection zone 13 containing the detection dry strip chemistry ingredients to the carrier strip 11. The carrier strip 11 contains an aperture 14 exposing the surface of the detection zone to the effect of atmospheric oxygen.

Fig. 2 is an isometric view showing the dry test strip of the invention having a plurality of oxygen providing apertures. The dry test strip comprises a carrier strip 21 having multiple apertures 24 positioned in the carrier strip. Applied to the surface of the carrier strip 21 surrounding the apertures 24 is a layer of pressure sensitive adhesive 22. The dry chemistry detection zone 23 is adhered to the carrier strip 21 through the pressure sensitive adhesive layer 22. The surface of the dry chemistry detection zone is apparent and is shown through the apertures 24.

Fig. 3 is an isometric view of the dry test strip of the invention. The dry test strip comprises a thermoplastic flat carrier strip 31 to which is applied the dry chemistry detection zone 33 which is adhered to the carrier strip 31 with an adhesive layer. Covering the detection zone 33 is a plastic film 32 which is adhered to the carrier strip with a pressure sensitive adhesive in the adhesion zone 35. In the protective film is an oxygen providing aperture 34 which provides atmospheric oxygen to the dry chemical strip detection chemistry components.

Fig. 4 is a cross-sectional side view of the dry test strip shown in Fig. 3. The dry test strip comprises a carrier strip 41 to which is attached, through a pressure sensitive adhesive layer 45, the detection zone containing the dry chemistry reagents 43. Covering the detection zone is a plastic film 42 which is adhered to the dry test strip at an additional adhesive layer 46. The carrier strip 41 has an aperture 47 for the purpose of delivering oxygen to the reagent system in the detection zone. Additionally the protective film has an oxygen providing aperture 44 that provides oxygen to the chemistry detection system in the detection zone 43.

Fig. 5 is a cross-sectional side view of a dry test strip of the invention. The dry test strip 50 comprises a carrier strip 51 to which is attached a detection zone 52. Between the detection strip 52 and the carrier strip 51 is an aperture 53 providing oxygen to the reverse carrier strip side of the detection zone. The aperture 53 is created by adhering the detection zone 52 to the carrier strip 51 using adhesive means 54 as spacers providing sufficient space or aperture between the carrier strip and the detection zone to permit the flow of adequate oxygen to the carrier side of the detection zone. In this embodiment, the carrier strip 51 may or may not have apertures formed in the plastic layer itself.

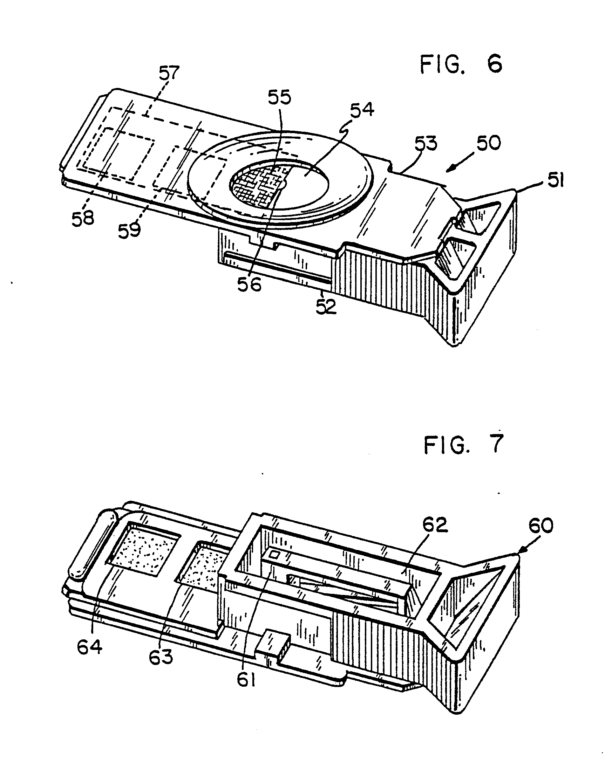

Fig. 6 is a view of a plastic machine read holder for the detection surface of the invention. The holder 50 comprises a handle 51 and a body 52 supporting a circular touch zone 53 having an internal opening 54. Exposed within the opening 54 is a wicking fabric 55 and a lance 56. The wicking fabric 55 is in fluid contact with the hidden dry test strip 57. The dry test strip 57 comprises a registration zone 58 and a detection zone 59 both of which are in fluid contact with the wicking fabric. As the registration zone 58 is wetted by the test fluid, technically blood, the appearance of the registration zone shows that full wetting has occurred. The detection zone 59 generates a color or other signals specifically indicating the concentration in a quantitative or semi-quantitative fashion of the target analyte in the blood serum.

Fig. 7 is a view of the opposite side of the holder of Fig. 5. The holder 60 contains a lance 61 mounted on a flexible support 62. In use the flexible support 62 is flexed, causing the lance 61 to penetrate through the surface of the holder shown in Fig. 6 at 66. The lance enters a finger to provide a blood sample. The holder 70 further comprises windows 73 and 74 which show a signal in the presence of the target analyte and the successful wetting of the dry test strip with the test fluid.

Example 1

A dry test strip of the invention was prepared by first preparing a methanol solution containing, in each 20 milliliters of the solution, 200 milligrams of orthotolidine. In addition an aqueous citrate-EDTA buffer solution at pH of 6 was prepared containing 6,000 units of glucose oxidase, 9,000 units of horseradish peroxidase, 0.5 wt-% of sodium dodecyl sulfonate, 20 wt-% of an 80,000 molecular weight dextran, 0.8 wt-% of a vinyl ether/maleic anhydride copolymer having a molecular weight of 20,000, 0.4 wt-% of a polyvinylpyrollidone polymer having a molecular weight of 40,000, 0.005 wt-% of ascorbic acid, and 0.75 wt % of tartrazine (FD&C yellow dye No. 5).

The dextran used in this Example was dialyzed against the citrate-EDTA buffer for 5 days, replacing the buffer once per day to remove mono, di and trisaccharide impurities.

A nylon membrane prepared by casting nylon on a nonwoven polyfabric (Ultipore 66, Pall) was first dipped in the citrate EDTA buffer solution, scraped, dried for 4 minutes at 75 degrees C, dipped in the methanolic orthotolidine solution. After drying at 75 degrees C. for 4 minutes, the membrane was dried and cut into a detection zone of dimensions approximately 6 by 6 millimeters. The zone was applied to a layer of pressure sensitive acrylic adhesive or a polyester substrate adhesive (3M#444) on a carrier strip having dimensions of 6.3 by 75 millimeters (0.2″ by 3″) containing a circular aperture in the carrier strip having a diameter of 5 millimeters (5/32″).

Example II

Example I was repeated exactly except that a dextran having a molecular weight of 35,600 was used.

Example III

Example I was repeated exactly except that a dextran having a molecular weight of 17,200 used.

Examples IV-VI

Examples I-III were repeated except that 0.6 K units of glucose oxidase and 0.9 K units of horseradish peroxidase were substituted for the 6 K units and 9 K units of glucose oxidase and horseradish peroxidase, respectively.

Discussion

Each of the test strips prepared in the Examples I-VI provided acceptable performance, while the test strip of Examples II and III provided a complete end point reading at about 1 minute and about 45 seconds after application of 15 u liters of whole blood.

Example VII

A dry test strip of the invention was made using the following procedure. A methanolic solution was prepared containing in each 10 milliliters of methanol, 50 milligrams of orthotoluidine, 10 milligrams of 3,3′,5,5′-tetramethyl benzidine, 0.25 wt-% sodium dodecyl sulfonate, 0.5 wt-% of a methyl vinyl ether/maleic anhydride copolymer having a molecular weight of about 20,000. Additionally an aqueous citrate EDTA buffer having a pH of about 6 was prepared containing, per each 10 milliliters of buffer, 6 K units of glucose oxidase and 9 K units of peroxidase. The EDTA buffer contained 20 wt-% of a 40,000 molecular weight dextran, 0.8 wt-% of the methyl vinyl ether/maleic anhydride copolymer, 0.4 wt-% of a polyvinylpyrolidone polymer having a molecular weight of 40,000, 0.005 wt-% of ascorbic acid, and 0.075 wt-% of tartrazine. Nylon membranes were first dipped into the citrate-EDTA buffer enzyme solution, scraped and dried for 4 minutes at 75 degrees C. and then dipped in the methanolic reagent solution, withdrawn and dried for 2 minutes at 75 degrees C. The nylon membrane was then divided into detection zone portions having dimensions of 6 by 6 millimeters. The detection zone was applied to a layer of pressure sensitive adhesive on the surface of a plastic carrier strip over an aperture having a diameter of 5 millimeters (5/32").

## Example VIII

Example VII was repeated exactly except that the methanol dip solution contained 0.1 wt-% sodium dodecyl sulfonate and 0.2 wt-% of the methyl vinyl ether/maleic anhydride copolymer.

## Example IX

Example VII was repeated exactly except that the methanol dip contained 0.1 wt-% of the sodium dodecyl sulfonate.

## Discussion

The dry test strips of the invention prepared in Examples VII through IX were tested by applying 20 microliters of whole blood containing in each application 20, 40, 80, 120, 160, 250 and 465 milligrams of glucose per each 100 milliliters of blood. In each evaluation, no red blood cell penetration was noted. The color signal was smooth and had no local concentrations. The optimum color signal was obtained in Example VII, while the colors in Examples VIII and IX appeared dark but could be discriminated at even the highest concentrations of glucose.

The foregoing detailed description of the invention is provided to illustrate and explain the dry test strip of the invention and to explain and illustrate its manufacture and use. While many embodiments of the invention can be made without departing from the spirit and scope of the invention, the invention resides in the claims hereinafter appended.

## Claims

1. A dry test strip for detection of an analyte in a test fluid utilizing a detection means requiring a concentration of atmospheric oxygen, which dry test strip comprises (a) a carrier strip, and (b) a detection zone containing a detection means requiring atmospheric oxygen, said zone having a carrier strip side attached to the carrier strip; wherein said dry test strip includes an aperture providing atmospheric oxygen to the carrier strip side of the detection zone.

2. A dry test strip as claimed in claim 1 wherein the detection zone is enclosed by a film which is also adhered to the carrier strip.

3. A dry test strip as claimed in claim 2 wherein the film contains an aperture that can provide access by atmospheric oxygen to the detection zone.

4. A dry test strip as claimed in any of claims 1 to 3 wherein the analyte is glucose.

5. A dry test strip as claimed in any of claims 1 to 4 wherein the carrier strip is a flexible thermoplastic material 25 to 300 millimetres in length, 1 to 10 millimeters in width, and 0.1 to 1 millimetres in thickness.

6. A dry test strip as claimed in claim 5 wherein the carrier strip has a circular aperture having a radius of about 3 to 10 millimeters.

7. A dry test strip as claimed in any of claims 1 to 6 wherein the carrier strip has a plurality of apertures.

8. A dry test strip as claimed in any of claims 1 to 7 wherein the film enclosure contains a plurality of apertures.

9. A dry test strip as claimed in any of claims 1 to 8 wherein the detection means comprises a chemical assay system or an immunoassay system.

10. A dry test strip as claimed in any of claims 1 to 9 wherein the carrier strip comprises a fabric.

11. A dry test strip as claimed in any of claims 1 to 10 wherein the detection zone comprises a matrix containing a labeled reagent zone containing a labeled antibody, a trapping zone containing immobilized antigen, and a detection zone wherein test fluid applied to the matrix penetrates the labeled reagent zone wherein any analyte present in the test fluid binds to the labeled antibody causing the labeled antibody antigen complex to pass the trapping zone and to penetrate into the detection zone, to generate a specific signal in proportion to the amount of analyte in the test fluid.

12. A dry test strip as claimed in any of claims 1 to 11 wherein the detection zone comprises a support made of a nylon matrix comprising a porous nylon membrane cast on a nonwoven thermoplastic fabric.

13. A dry test strip as claimed in any of claims 1 to 12 wherein the test fluid comprises whole blood.

14. A method for the detection of an analyte in a test fluid which comprises applying a test fluid to a dry test strip as claimed in any of claims 1 to 13, and reading the detection signal produced by the detection zone in response to the concentration of analyte in the test fluid.

FIG. IA   FIG. IB   FIG. IC

FIG. 2A   FIG. 2B   FIG. 2C

FIG. 3   FIG. 4   FIG. 5

FIG. 6

FIG. 7